# EUROPEAN PATENT APPLICATION

(11) **EP 2 902 016 A1**
(43) Date of publication of application: **05.08.2015**
(21) Application number: 15000227.7
(22) Date of filing: 27.01.2015
(51) Int. Cl.: A61K 9/20, A61K 31/426

(54) **Febuxostat tablet**

(30) Priority: 30.01.2014 IN DE02772014
(71) Applicant: Alfred E. Tiefenbacher (GmbH & Co. KG), 22767 Hamburg (DE)
(72) Inventor: Gujjar, Chaitanya Yogananda, 577 202 Shimoga - Karnataka (IN); Rallabandi, Bala Ramesha Chary, Hyderabad - 500 090 Andhra Pradesh (IN); Patwari, Pradip Shivraj, Latur - 413 517 Maharashtra (IN)

(57) **Abstract**

The present invention relates to a tablet containing febuxostat in crystalline form, which is prepared by granulation. The tablet contains the intragranular and extragranular pharmaceutical excipients in a specific weight ratio, and optionally a surfactant.

## Description

The present invention relates to a tablet for oral administration containing febuxostat, wherein the tablet is prepared using a granulation process.

Febuxostat is a xanthine oxidase inhibitor useful in decreasing serum uric acid. Febuxostat is marketed under the trade name Uloric^{®} or Adenuric^{®} as a film-coated tablet containing 40 mg, 80 mg or 120 mg febuxostat. The tablet is approved for the chronic management of hyperuricemea in patients with gout.

According to the Biopharmaceutics Classification System febuxostat is classified as a class 2 compound (low solubility, high permeability). Febuxostat is practically insoluble in water, which results in a low bioavailability following oral administration. In addition, it is commonly known that various crystalline forms of febuxostat exist. EP 1 020 454 describes the crystalline forms A, B, C, D and G of febuxostat, while WO 2010/144685 discloses the crystalline forms F1-F14 and WO 2008/067773 pertains to the crystalline forms H, I and J. These crystalline forms may interconvert during manufacture or storage of the solid dosage form. However, different crystalline forms of a drug may show different dissolution properties, so that it is difficult to obtain a febuxostat containing tablet having no variation in the drug's dissolution profile. Various approaches have been suggested in the prior art in order to overcome the dissolution and, thus, bioavailability problems of febuxostat.

EP 1 488 790 discloses a tablet containing the crystalline form A of febuxostat, which is prepared by wet-granulation. A mixture containing the crystalline form A of febuxostat, lactose, pregelatinized starch and hydroxypropyl cellulose (HPC) was subjected to granulation with water. The obtained granules were mixed with croscarmellose sodium and magnesium stearate, and the mixture was compressed into tablets.

WO 2012/172461 pertains to a tablet comprising febuxostat, which is prepared by wet-granulation. The dissolution rate and bioavailability of the drug is improved by using febuxostat fine particles having an average particle size of from 1 µm to 50 µm.

CN 101474175 discloses a tablet containing febuxostat, preferably the crystalline forms C, H, I, J or mixtures thereof, in which the drug has an average particle size of 3.5 µm to 10 µm.

WO 2012/153313 relates to a tablet, which is prepared by applying a suspension containing febuxostat in micronized form having an average particle size of less than 50 µm, a hydrophilic polymer and optionally a surfactant to an inert carrier. The drug coated granules are subsequently compressed into tablets.

WO 2012/140632 discloses the use of febuxostat in a non-crystalline form for the preparation of a pharmaceutical composition, e.g. a tablet. It is reported that a solid dispersion of febuxostat within a carrier, preferably polyvinylpyrrolidone, is stable upon storage under ambient conditions.

IN 2116/MUM/2010 discloses a tablet containing febuxostat and a solubility enhancing agent, which is preferably selected from a surfactant and polyvinylpyrrolidone. The tablet is prepared by wet-granulation, whereby a mixture containing febuxostat and pharmaceutical excipients is subjected to a wet-granulation with an aqueous granulation liquid containing polyvinylpyrrolidone and optionally sodium lauryl sulfate. The average particle size of febuxostat is from 1 µm to less than 12 µm, preferably about 10 µm.

WO 2013/001441 pertains to a tablet containing febuxostat using dry processes, i.e. dry granulation or direct compression. The average particle size of febuxostat is from 1 µm to 50 µm.

Hence, various formulations have been suggested in the state of the art, which provide a sufficiently high dissolution rate to febuxostat. The problem of increasing the dissolution rate of febuxostat has been solved by micronizing the drug, using solubility enhancing agents and disintegrants. It was the objective of the present invention to provide an alternative tablet for oral administration containing febuxostat.

This objective is attained by the subject matter as defined in the claims.

The present invention relates to a tablet containing febuxostat prepared by dry-granulation or wet-granulation. It has been found that the weight ratio of the pharmaceutical excipients contained intragranularly to the pharmaceutical excipients contained extragranularly has an impact on the dissolution profile of febuxostat. The dissolution rate of the drug is increased, if the amount of the pharmaceutical excipients used for preparing the febuxostat containing granules is decreased, while the amount of the pharmaceutical excipients, which are mixed with the granules and subsequently compressed into tablets, is increased. Accordingly, the present invention relates to a process for preparing a tablet for oral administration containing febuxostat in crystalline form, comprising the steps of:
i) subjecting a mixture containing febuxostat and a first pharmaceutical excipient to dry granulation or wet granulation with a granulation liquid,
ii) mixing the granulate obtained in step (i) with a second pharmaceutical excipient,
iii) subjecting the mixture obtained in step (ii) to compression to obtain a tablet, and optionally
iv) subjecting the tablet obtained in step (iii) to film coating,
wherein the weight ratio of the second pharmaceutical excipient to the first pharmaceutical excipient is at least 1 : 2. According to a preferred embodiment of the present invention, the weight ratio of the second pharmaceutical excipient to the first pharmaceutical excipient is at least 1 : 1, preferably from 1 : 1 to 3.5 : 1. As a result of decreasing the amount of the pharmaceutical excipient in the preparation of the granules, the weight ratio of febuxostat to the first pharmaceutical excipient is increased. Accordingly, the present invention relates to a process, in which the granulation may afford granules containing febuxostat and the first pharmaceutical excipient in a weight ratio of at least 1 : 5, preferably at least 1 : 2.5, more preferred from 1 : 2.5 to 1.5 : 1, and most preferred from 1 : 2.5 to 1 : 1. In an even more preferred embodiment of the present invention, the weight ratio of febuxostat to the first pharmaceutical excipient is from 1 : 2.5 to 1 : 1, while the weight ratio of the second pharmaceutical excipient to the first pharmaceutical excipient is from 1 : 1 to 3.5 : 1.

It has been found that the use of a surfactant as extragranular component in the tablet of the present invention may further improve the dissolution of febuxostat. Moreover, this measure also minimizes any variations of the dissolution profile originating from the different solubilities of febuxostat's crystalline forms. Hence, in the process of the present invention the second pharmaceutical excipient preferably comprises a surfactant.

Examples of surfactants which can be used in the process of the present invention include anionic, cationic and non-ionic surfactants, e.g. sodium lauryl sulfate, poloxamers and polysorbates. The use of sodium lauryl sulfate is particularly preferred in the present invention.

In the process of the present invention, it is preferred that the first pharmaceutical excipient and the second pharmaceutical excipient comprise a disintegrant. The disintegrants used as intragranular and extragranular components may be the same or different and may be selected from croscarmellose sodium, sodium starch glycolate, polyvinylpolypyrrolidone (crospovidone) and low substituted hydroxypropyl cellulose (L-HPC). Preferably, the disintegrants are selected from croscarmellose sodium and L-HPC.

The present invention also relates to a tablet obtained by the process mentioned above. Preferably, the tablet or, if film-coated, the tablet core consists of:
- intragranular: febuxostat, a first diluent, a binder, a first disintegrant and a first lubricant, and
- extragranular: a second diluent, a second disintegrant, a second lubricant, a glidant and a surfactant.

Examples of the diluent include microcrystalline cellulose, calcium hydrogen phosphate, lactose (anhydrous or monohydrate), mannitol, sorbitol and calcium carbonate.

Examples of the binder include methyl cellulose, hydroxypropylmethyl cellulose (HPMC), hydroxypropyl cellulose (HPC), polyethylene glycol, maltodextrin, pregelatinized starch, polyvinylpyrrolidone (povidone) and vinylpyrrolidone/vinylacetate copolymer (copovidone).

As glidants silicone dioxide, talk and the like may be used, while magnesium stearate, calcium stearate, stearic acid, sodium stearyl fumarate and glycerol dibehenate are examples of suitable lubricants. Preferably, the glidant is colloidal silicone dioxide, and the lubricant is magnesium stearate.

According to a preferred embodiment of the present invention, the first diluent is selected from microcrystalline cellulose and mannitol, the second diluent is selected from microcrystalline cellulose, mannitol and calcium carbonate, the binder is selected from HPC and maize starch, the first and the second disintegrants are selected from L-HPC and croscarmellose sodium. More preferably, the second disintegrant is croscarmellose sodium.

The tablet of the present invention may contain an alkalizing agent. Examples of the alkalizing agent include potassium bicarbonate, potassium citrate, sodium bicarbonate, sodium citrate dihydrate, sodium carbonate, magnesium oxide and lysine.

It has been found that the tablet according to the present invention preferably contains febuxostat in the crystalline form G as described in EP 1 020 454 or F10 as disclosed in WO 2010/144685.

According to a preferred embodiment of the present invention, the febuxostat is contained in the tablet in micronized form. Milling or micronization techniques that may be used for particle size reduction include sifting, milling by, e.g., ball, roller, and hammer mills, as well as jet mills. The particle size distribution of febuxostat particles may be measured using any technique known in the art, e.g. by microscopy or light scattering equipment such as a Malvern Mastersizer 2000. The particle size distributions can be expressed in terms of D(90), D(50) and D(10) values, where the values (expressed in µm) are the maximum sizes for 90, 50 and 10 % of the particles, respectively. A micronized febuxostat, which can be employed in the present invention, has a particle size distribution, according to which D(90) is equal or less than 25 µm, preferably ≤ 20 µm, more preferred ≤ 15 µm and most preferred ≤ 5 µm. The D(50) value is equal or less than 15 µm, preferably ≤ 10 µm, more preferred ≤ 5 µm and most preferred ≤ 1.5 µm. The D(10) value is equal or less than 5 µm, preferably ≤ 2.5 µm, more preferred ≤ 1.5 µm and most preferred ≤ 1.0 µm. Hence, preferred is the use of a micronized febuxostat having a particle size contribution of D(90) ≤ 15 µm, D(50) ≤ 5 µm and D(10) ≤ 1.5 µm and most preferred is the use of a micronized febuxostat having a particle size contribution of D(90) ≤ 5 µm, D(50) ≤ 1. 5 µm and D(10) ≤ 1.0 µm.

### Examples

The tablets described in the examples and comparative examples contain either the form G or the form F10 of febuxostat in micronized form (Mastersizer 2000): Form G: D(90) = 4.2 µm, D(50) = 1.5 µm and D(10) = 0.5 µm; Form F10 D(90) = 13.6 µm, D(50) = 3.5 µm and D(10) = 1.0. The dissolution profile was determined according to the dissolution test for solid dosage forms described in chapter 2.9.3 of the European Pharmacopeia 7.8 using acetate buffer (pH 4.5, 1.0 % w/v Tween 80, 1000 ml, apparatus: paddle, 50 rpm), phosphate buffer (pH 6.8, 900 ml, apparatus: paddle, 75 rpm) or phosphate buffer (pH 6.0, 900 ml, apparatus: paddle, 75 rpm) at 37 ± 0.5°C. The used film coating system Opadry^{®} II Green 85F510033 comprises polyvinyl alcohol, titanium dioxide (E 171), D&C Yellow # 10 Aluminum Lake, FD&C Blue # 1/ Brilliant Blue FCF Aluminum Lake, macrogol and talc.

**Comparative Examples 1-5**

| **Ingredients** | **Comp. Ex. 1** | **Comp. Ex. 2** | **Comp. Ex. 3** | **Comp. Ex. 4** | **Comp. Ex. 5** |
|---|---|---|---|---|---|
| | **Milligram /Tablet** | | | | |
| **Stage - A (Dry mix, Blending & Compaction)** | | | | | |
| Febuxostat Form G | 82.51 | 82.51 | 82.51 | 80.00 | - |
| Febuxostat Form F10 | - | - | - | - | 80.00 |
| Cellulose, Microcrystalline (Avicel^{®} PH102) | - | 298.49 | 303.49 | - | - |
| Cellulose, Microcrystalline (Comprecel^{®} M 102D+) | 300.49 | - | - | 189.30 | 189.30 |
| Lactose Monohydrate (Tablettose^{®} 80) | 76.50 | 76.50 | 76.50 | 178.30 | 178.30 |
| Croscarmellose Sodium (Ac-Di-Sol^{®}) | 20.00 | - | - | 25.00 | 25.00 |
| Low substituted Hydroxypropyl cellulose (L-HPC LH31) | - | 25.00 | - | - | - |
| Crospovidone Type B (Polyplasdone XL-10) | - | - | 20.00 | - | - |
| Hydroxypropyl Cellulose (Klucel^{®} LF Pharma) | 10.00 | 10.00 | 10.00 | - | - |
| Hydroxypropyl Cellulose (Klucel^{®} EXF Pharma) | - | - | - | 15.40 | 15.40 |
| Magnesium Stearate | 4.00 | 2.50 | 2.50 | | |
| Silica, Colloidal Anhydrous (Aerosil^{®} 200) | - | - | - | 4.80 | 4.80 |

| **Stage - B (Blending & Lubrication)** | | | | | |
|---|---|---|---|---|---|
| Silica, Colloidal Anhydrous (Aerosil^{®} 200) | 2.50 | 2.50 | 2.50 | - | - |
| Magnesium Stearate | 4.00 | 2.50 | 2.50 | 7.20 | 7.20 |
| ***Weight of core tablets*** | 500.00 | 500.00 | 500.00 | 500.00 | 500.00 |

| **Stage - C (Coating)** | | | | | |
|---|---|---|---|---|---|
| Opadry^{®} II Green 85F510033 | 16.00 | 16.00 | 16.00 | 16.00 | 16.00 |
| Water, Purified | Q.s. | Q.s. | Q.s. | Q.s. | Q.s. |
| ***Weight of coated tablets*** | 516.00 | 516.00 | 516.00 | 516.00 | 516.00 |

### Manufacturing Procedure:

Febuxostat and the excipients of stage A were sifted and subjected to compaction. The compacted material was subsequently milled, sifted and blended with Magnesium stearate and optionally silica. The mixture was subjected to compression to obtain a tablet, which was finally film-coated.

**Comparative Dissolution Profiles of Febuxostat Film-coated Tablets 80 mg with Uloric^{®} 80 mg in 1.0 % Tween 80 in Acetate buffer solution pH 4.5**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 60 | 77 | 84 | 91 | 95 | 99 | 101 |
| Comp. Ex. 1 | 46 | 56 | 63 | 67 | 72 | 77 | 80 |
| Comp. Ex. 2 | 53 | 67 | 74 | 78 | 83 | 90 | 91 |
| Comp. Ex. 3 | 49 | 62 | 68 | 75 | 81 | 86 | 90 |

**Comparative Dissolution Profiles of Febuxostat Film-coated Tablets 80 mg with Uloric^{®} 80 mg in Phosphate Buffer solution pH 6.8**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 85 | 97 | 98 | 98 | 98 | 99 | 99 |
| Comp. Ex. 1 | 84 | 90 | 92 | 93 | 94 | 95 | 96 |
| Comp. Ex. 4 | 89 | 95 | 96 | 96 | 97 | 97 | 97 |
| Comp. Ex. 5 | 92 | 95 | 96 | 97 | 97 | 97 | 98 |

**Comparative Dissolution Profiles of Febuxostat Film-coated Tablets 80 mg with Uloric^{®} 80 mg in Phosphate Buffer solution pH 6.0**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 76 | 85 | 90 | 95 | 97 | 97 | 98 |
| Comp. Ex. 4 | 61 | 73 | 79 | 83 | 87 | 90 | 92 |
| Comp. Ex. 5 | 79 | 87 | 91 | 94 | 96 | 98 | 99 |

### Observations

The dissolution profile of Comparative Examples 1 to 4 was not sufficient. The flow properties of the blends of Comparative Examples 4 and 5 were very poor. The compressibility index of Comparative Example 4 was 39.474% and that of Comparative Example 5 was 36.765%. During compression of the blends of Comparative Examples 4 and 5 heavy sticking was observed on tablet and punch surface.

**Examples 1 to 4**

| **Ingredients** | **Ex. 1** | **Ex. 2** | **Ex. 3** | **Ex. 4** |
|---|---|---|---|---|
| | **Milligram /Tablet** | | | |
| **Stage - A (Dry mix, Blending & Compaction)** | | | | |
| Febuxostat Form G | 82.51 | 82.51 | 82.51 | 82.51 |
| Cellulose, Microcrystalline (Avicel 102) | 10.00 | 30.00 | 10.00 | 20.00 |
| Lactose Monohydrate (Tablettose^{®} 80) | 26.50 | 26.50 | 26.50 | 26.50 |
| Croscarmellose Sodium (Ac-Di-Sol^{®}) | 30.00 | 10.00 | - | - |
| Low substituted Hydroxypropyl cellulose (L-HPC LH31) | - | - | 30.00 | 20.00 |
| Magnesium Stearate | 2.00 | 2.00 | 2.00 | 2.00 |

| **Stage - B (Lubrication & Blending)** | | | | |
|---|---|---|---|---|
| Cellulose, Microcrystalline (Avicel 102) | 323.49 | 323.49 | 333.49 | 333.49 |
| Croscarmellose Sodium (Ac-Di-Sol^{®}) | 10.00 | 10.00 | - | - |
| Low substituted Hydroxypropyl cellulose (L-HPC LH31) | - | - | 10.00 | 10.00 |
| Hydroxypropyl Cellulose (Klucel^{®} EXF Pharma) | 10.00 | 10.00 | - | - |
| Colloidal Silicon dioxide (Aerosil^{®} 200) | 2.50 | 2.50 | 2.50 | 2.50 |
| Magnesium Stearate | 3.00 | 3.00 | 3.00 | 3.00 |
| ***Weight of core tablets*** | **500.00** | **500.00** | **500.00** | **500.00** |

| **Stage - C (Coating)** | | | | |
|---|---|---|---|---|
| Opadry^{®} Green 85F510033 | 16.00 | 16.00 | 16.00 | 16.00 |
| Water, Purified | Q.s. | Q.s. | Q.s. | Q.s. |
| ***Weight of coated tablets*** | **516.00** | **516.00** | **516.00** | **516.00** |

### Manufacturing Procedure:

Febuxostat and the excipients of stage A were sifted and subjected to compaction. The compacted material was subsequently milled, sifted and blended with the excipients of stage B. The mixture was subjected to compression to obtain a tablet, which was finally film-coated.

**Comparative Dissolution Profiles of Febuxostat Film-coated Tablets 80 mg with Uloric^{®} 80 mg in 1.0% Tween 80 in Acetate buffer solution pH 4.5**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 60 | 77 | 84 | 91 | 95 | 99 | 101 |
| Example 1 | 55 | 67 | 75 | 81 | 86 | 90 | 94 |
| Example 2 | 46 | 60 | 68 | 75 | 81 | 86 | 90 |
| Example 3 | 63 | 76 | 85 | 89 | 95 | 99 | 100 |
| Example 4 | 63 | 77 | 84 | 86 | 93 | 93 | 99 |

**Examples 5-8**

| **Ingredients** | **Ex. 5** | **Ex. 6** | **Ex. 7** | **Ex. 8** |
|---|---|---|---|---|
| | **Milligram /Tablet** | | | |
| **Stage-A (Dry mix & Compaction)** | | | | |
| Febuxostat Form G | 80.00 | - | 80.00 | - |
| Febuxostat Form F10 | - | 80.00 | - | 80.00 |
| Low Substituted Hydroxypropyl Cellulose (L-HPC LH 31) | 30.00 | - | - | 30.00 |
| Cellulose, Microcrystalline (Comprecel^{®} M 102 D+) | - | 117.16 | 117.16 | - |
| Hydroxypropyl cellulose (Klucel^{®} EXF Pharma) | - | 10.00 | 10.00 | - |
| Croscarmellose sodium (Ac-Di-Sol^{®}) | - | 10.00 | 10.00 | - |
| Mannitol (Pearlitol^{®} SD 200) | 46.50 | 30.00 | 30.00 | 46.50 |
| Maize, starch | 20.00 | - | - | 20.00 |
| Magnesium Stearate | 2.00 | 2.00 | 2.00 | 2.00 |

| **Stage-B (Blending & Lubrication)** | | | | |
|---|---|---|---|---|
| Cellulose, Microcrystalline (Comprecel^{®} M 102 D+) | 232.00 | 181.84 | 181.84 | 232.00 |
| Croscarmellose sodium (Ac-Di-Sol^{®}) | 10.00 | 10.00 | 10.00 | 10.00 |
| Mannitol (Pearlitol^{®} SD 200) | 50.00 | 46.50 | 46.50 | 50.00 |
| Sodium Laurilsulfate | 4.00 | 2.00 | 2.00 | 4.00 |
| Calcium Carbonate | 20.00 | - | - | 20.00 |
| Colloidal Silicon dioxide (Aerosil^{®} 200 pharma) | 2.50 | 2.50 | 2.500 | 2.50 |
| Magnesium Stearate | 3.00 | 8.00 | 8.00 | 3.00 |
| ***Weight of core tablet*** | **500.00** | **500.00** | **500.00** | **500.00** |

| **Stage-C (Coating)** | | | | |
|---|---|---|---|---|
| Opadry® II Green 85F510033 | 16.00 | 16.00 | 16.00 | 16.00 |
| Water, Purified | Qs | Qs | Qs | Qs |
| ***Weight of coated tablets*** | **516.00** | **516.00** | **516.00** | **516.00** |

### Manufacturing Procedure:

Febuxostat and the excipients of stage A were sifted and subjected to compaction. The compacted material was subsequently milled, sifted and blended with the excipients of stage B. The mixture was subjected to compression to obtain a tablet, which was finally film-coated.

**Comparative Dissolution Profiles of Febuxostat Film-coated Tablets 80 mg with Uloric^{®} 80 mg in Phosphate Buffer solution pH 6.8**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 85 | 97 | 98 | 98 | 98 | 99 | 99 |
| Example 5 | 95 | 96 | 97 | 97 | 98 | 97 | 99 |
| Example 6 | 98 | 99 | 98 | 98 | 98 | 98 | 98 |
| Example 7 | 93 | 96 | 97 | 98 | 98 | 98 | 98 |
| Example 8 | 95 | 96 | 96 | 97 | 97 | 97 | 97 |

**Comparative Dissolution Profiles of Febuxostat Film-coated Tablets 80 mg with Uloric^{®} 80 mg in Phosphate Buffer solution pH 6.0**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 76 | 85 | 90 | 95 | 97 | 97 | 98 |
| Example 5 | 87 | 91 | 95 | 97 | 98 | 99 | 98 |
| Example 6 | 78 | 86 | 90 | 92 | 94 | 95 | 96 |
| Example 7 | 73 | 80 | 85 | 88 | 91 | 94 | 95 |
| Example 8 | 90 | 95 | 97 | 97 | 98 | 98 | 98 |

**Examples 9 and 10**

| **Ingredients** | **Ex. 9** | **Ex. 10** |
|---|---|---|
| | **Milligram /Tablet** | |
| **Stage-A (Dry mix & Compaction)** | | |
| Febuxostat Form G | 80.00 | - |
| Febuxostat Form F10 | - | 80.00 |
| Low Substituted Hydroxypropyl Cellulose (L-HPC LH31) | 22.00 | - |
| Cellulose, Microcrystalline (Avicel^{®} PH 200) | - | 118.00 |
| Hydroxypropyl cellulose (Klucel^{®} EXF Pharma) | - | 5.00 |
| Croscarmellose sodium (Ac-Di-Sol^{®}) | - | 15.00 |
| Mannitol (Pearlitol^{®} SD 200) | 46.50 | 30.00 |
| Maize, starch | 10.00 | - |
| Magnesium Stearate | 2.00 | 2.00 |

| **Stage-B (Milling & Blending)** | | |
|---|---|---|
| Cellulose, Microcrystalline (Comprecel^{®} M 102 D+) | 242.00 | - |
| Cellulose, Microcrystalline (Avicel^{®} PH 200) | - | 173.50 |
| Croscarmellose sodium (Ac-Di-Sol^{®}) | 10.00 | 10.00 |
| Sodium Laurilsulfate | 2.00 | 2.00 |
| Calcium Carbonate | 20.00 | - |
| Colloidal Silicon dioxide (Aerosil^{®} 200 pharma) | 2.50 | 5.00 |

| **Stage-C (Blending)** | | |
|---|---|---|
| Mannitol (Pearlitol^{®} SD 200) | 50.00 | 46.50 |

| **Stage-D (Lubrication)** | | |
|---|---|---|
| Magnesium Stearate | 13.00 | 13.00 |
| ***Weight of core tablet*** | **500.00** | **500.00** |

| **Stage-E (Coating)** | | |
|---|---|---|
| Opadry^{®} II Green 85F510033 | 16.00 | 16.00 |
| Water, Purified | Qs | Qs |
| ***Weight of coated tablets*** | **516.00** | **516.00** |

### Manufacturing Procedure:

Febuxostat and the excipients of stage A were sifted and subjected to compaction. The compacted material was subsequently milled, sifted and blended with the excipients of stage B. The mixture was blended with the excipients of stage C and further lubricated with the excipients of stage D. The mixture was subjected to compression to obtain a tablet, which was finally film-coated.

**Comparative Dissolution Profiles of Febuxostat Film-coated Tablets 80 mg with Uloric^{®} 80 mg in Phosphate Buffer solution pH 6.8**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 85 | 97 | 98 | 98 | 98 | 99 | 99 |
| Example 9 | 95 | 97 | 97 | 97 | 98 | 98 | 99 |
| Example 10 | 94 | 95 | 95 | 95 | 95 | - | - |

**Comparative Dissolution Profiles of Febuxostat Film-coated Tablets 80 mg with Uloric^{®} 80 mg in Phosphate Buffer solution pH 6.0**

| **Product** | **Mean Cumulative % Labeled Amount Dissolved** | | | | | | |
|---|---|---|---|---|---|---|---|
| | **5 min** | **10 min** | **15 min** | **20 min** | **30 min** | **45 min** | **60 min** |
| Uloric^{®} 80 mg | 76 | 85 | 90 | 95 | 97 | 97 | 98 |
| Example 9 | 78 | 89 | 92 | 94 | 95 | 97 | 97 |
| Example 10 | 74 | 86 | 89 | 92 | 95 | - | - |

**Examples 11 and 12**

| **Ingredients** | **Ex. 11** | **Ex. 12** |
|---|---|---|
| | **Milligram /Tablet** | |
| **Stage-A (Dry mix & Compaction)** | | |
| Febuxostat Form G | 80.00 | 82.26 |
| Low Substituted Hydroxypropyl Cellulose (L-HPC LH31) | 22.00 | 30.00 |
| Mannitol (Pearlitol^{®} SD 200) | 46.50 | 31.50 |
| Maize, starch | 10.00 | - |
| Meglumine | - | 15.00 |
| Magnesium Stearate | 2.00 | 2.00 |

| **Stage-B (Milling & Blending)** | | |
|---|---|---|
| Cellulose, Microcrystalline (Comprecel^{®} M 102 D+) | 242.00 | - |
| Cellulose, Microcrystalline (Avicel^{®} PH 200) | - | 268.74 |
| Croscarmellose sodium (Ac-Di-Sol^{®}) | 10.00 | 10.00 |
| Low Substituted Hydroxypropyl Cellulose (L-HPC LH31) | - | 10.00 |
| Sodium Laurilsulfate | 2.00 | - |
| Meglumine | 15.00 | - |
| Mannitol (Pearlitol^{®} SD 200) | 55.00 | 45.00 |
| Colloidal Silicon dioxide (Aerosil^{®} 200 pharma) | 2.50 | 2.50 |
| Magnesium Stearate | 13.00 | 3.00 |
| ***Weight of core tablet*** | **500.00** | **500.00** |

| **Stage-C (Coating)** | | |
|---|---|---|
| Opadry^{®} II Green 85F510033 | 16.00 | 16.00 |
| Water, Purified | Qs | Qs |
| ***Weight of coated tablets*** | **516.00** | **516.00** |

### Manufacturing Procedure:

Febuxostat and the excipients of stage A were sifted and subjected to compaction. The compacted material was subsequently milled, sifted and blended with the excipients of stage B. The mixture was subjected to compression to obtain a tablet, which was finally film-coated.

**Example 13**

| **Ingredients** | **Ex. 13** |
|---|---|
| | **Milligram /Tablet** |
| **Stage-A (Dry mix & Compaction)** | |
| Febuxostat Form F10 | 80.00 |
| Cellulose, Microcrystalline (Avicel^{®} PH 200) | 118.00 |
| Hydroxypropyl cellulose (Klucel^{®} EXF Pharma) | 5.00 |
| Croscarmellose sodium (Ac-Di-Sol^{®}) | 22.60 |
| Mannitol (Pearlitol^{®} SD 200) | 30.00 |
| Magnesium Stearate | 2.00 |

| **Stage-B (Milling & Blending)** | |
|---|---|
| Cellulose, Microcrystalline (Avicel^{®} PH 200) | 145.74 |
| Croscarmellose sodium (Ac-Di-Sol^{®}) | 10.00 |
| Sodium Laurilsulfate | 2.00 |
| Colloidal Silicon dioxide (Aerosil^{®} 200 pharma) | 5.00 |

| **Stage-C (Blending)** | |
|---|---|
| Mannitol (Pearlitol^{®} SD 200) | 66.66 |

| **Stage-D (Lubrication)** | |
|---|---|
| Magnesium Stearate | 13.00 |
| ***Weight of core tablet*** | **500.00** |

| **Stage-E (Coating)** | |
|---|---|
| Opadry^{®} II Green 85F510033 | 16.00 |
| Water, Purified | Qs |
| ***Weight of coated tablets*** | **516.00** |

### Manufacturing Procedure:

Febuxostat and the excipients of stage A were sifted and subjected to compaction. The compacted material was subsequently milled, sifted and blended with the excipients of stage B. The mixture was blended with the excipients of stage C and further lubricated with the excipients of stage D. The mixture was subjected to compression to obtain a tablet, which was finally film-coated.

## Claims

1. A process for preparing a tablet for oral administration containing febuxostat in crystalline form, comprising the steps of:
i) subjecting a mixture containing febuxostat and a first pharmaceutical excipient to dry granulation or wet granulation with a granulation liquid,
ii) mixing the granulate obtained in step (i) with a second pharmaceutical excipient,
iii) subjecting the mixture obtained in step (ii) to compression to obtain a tablet, and optionally
iv) subjecting the tablet obtained in step (iii) to film coating,
wherein the weight ratio of the second pharmaceutical excipient to the first pharmaceutical excipient is at least 1 : 2.

2. Process according to claim 1, wherein the weight ratio of the second pharmaceutical excipient to the first pharmaceutical excipient is at least 1 : 1, preferably from 1 : 1 to 3.5 : 1.

3. Process according to claim 1 or 2, wherein the weight ratio of febuxostat to the first pharmaceutical excipient is at least 1 : 5, preferably at least 1 : 2.5, more preferably from 1 : 2.5 to 1.5 : 1, most preferably from 1 : 2.5 to 1 : 1.

4. Process according to claim 3, wherein the weight ratio of febuxostat to the first pharmaceutical excipient is from 1 : 2.5 to 1 : 1, and wherein the weight ratio of the second pharmaceutical excipient to the first pharmaceutical excipient is from 1 : 1 to 3.5 : 1.

5. Process according to any one of the preceding claims, wherein the second pharmaceutical excipient comprises a surfactant.

6. Process according to claim 5, wherein the surfactant is selected from sodium lauryl sulfate, poloxamers and polysorbates.

7. Process according to any one of the preceding claims, wherein the first pharmaceutical excipient and the second pharmaceutical excipient comprise a disintegrant.

8. Process according to any one of the preceding claims, wherein the crystalline form of febuxostat is the crystalline form G or F10.

9. A tablet obtained by a process according to any one of claims 1 to 8.

10. Tablet according to claim 9, which is optionally film-coated, wherein the tablet or tablet core consists of:
- intragranular: febuxostat, a first diluent, a binder, a first disintegrant and a first lubricant, and
- extragranular: a second diluent, a second disintegrant, a second lubricant, a glidant and a surfactant.

11. Tablet according to claim 10, wherein the first diluent is selected from microcrystalline cellulose and mannitol, the second diluent is selected from microcrystalline cellulose, mannitol and calcium carbonate, the binder is selected from hydroxypropyl cellulose (HPC) and maize starch, the first and the second disintegrants are selected from low substituted hydroxypropyl cellulose (L-HPC) and croscarmellose sodium.

12. Tablet according to claim 11, wherein the second disintegrant is croscarmellose sodium.

13. Tablet according to any one of claims 9 to 12, wherein the surfactant is sodium lauryl sulfate.

14. Tablet according to any one of claims 9 to 13, wherein the crystalline form of febuxostat is the crystalline form G or F10.
